(19) Europäisches Patentamt

European Patent Office

Office européen des brevets

(11) EP 3 081 162 A2

(12) **EUROPEAN PATENT APPLICATION**

(43) Date of publication:
19.10.2016 Bulletin 2016/42

(21) Application number: 16159904.8

(22) Date of filing: 11.03.2016

(51) Int Cl.:
*A61B 5/11* (2006.01)  *A61B 5/0295* (2006.01)
*A61B 5/053* (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 24.03.2015 US 201562137305 P

(71) Applicant: Covidien LP
Mansfield, MA 02048 (US)

(72) Inventor: Vogel, Jeffrey
Santa Rosa, CA 95403 (US)

(74) Representative: Gray, James
Withers & Rogers LLP
4 More London Riverside
London SE1 2AU (GB)

(54) **VASCULAR DISEASE DETECTION DEVICE**

(57) Devices, systems, and techniques for monitoring vascular disease of a user are disclosed. In some examples, a wearable device for monitoring chronic venous insufficiency of a user may include a band configured to couple the wearable device to a leg of a user, a strain sensor configured to generate strain information indicative of changes in circumference of at least a portion of the leg, an activity sensor configured to generate activity information indicative of one or more of an orientation or a movement of the leg, and a processor configured to output user information indicative of the strain information and the activity information.

FIG. 1

## Description

### RELATED APPLICATIONS

This Application claims the benefit of U.S. Provisional Application No. 62/137,305 filed on March 24, 2015, entitled "VASCULAR DISEASE DETECTION DEVICE", which is herein incorporated by reference in its entirety.

### TECHNICAL FIELD

The disclosure relates to medical devices and, more particularly, medical devices for detecting vascular disease and related conditions.

### BACKGROUND

Vascular disease affects many people and can result in blood flow related conditions, pain, and disabilities in some cases. For example, in the venous system, chronic venous insufficiency (CVI) is a condition in which blood is not adequately returned from the legs to the heart of a patient. CVI can be caused by one or more of: malfunctioning valves in one or more veins, weakened vessel walls, poor calf muscle pump function, or an obstruction such as a blood clot. Symptoms of CVI can include leg aches and cramping, itching, pain that worsens when standing, swelling of the legs, skin discoloration around the ankles, varicose veins, and skin conditions such as ulcers or hardening. Treatments for CVI may include leg compression stockings, increased physical activity, weight loss, or more invasive procedures such as sclerotherapy, ablation, or stenting for advanced progression of the disease.

In some cases, a physician may diagnose a patient with, for example, CVI based on visual inspection of the patient's legs during a routine exam and/or evaluating patient risk factors. In some cases, a physician may employ additional diagnostic techniques such as duplex ultrasound, plethysmography, or other minimally invasive imaging methods. However, each of these tests requires administration by one or more healthcare professionals in an office or hospital setting. The tests may therefore not be performed during the normal course of life and routine of the patient, and, therefore, may not capture a full set of information useful for diagnosing CVI. In addition, these diagnostic methods may rely upon a single measurement made at one point in time instead of a larger pool of data obtained over a larger duration of time that may be more reflective of the actual patient condition as experienced during the normal course of life.

### SUMMARY

In general, the present disclosure provides methods and devices to detect CVI by monitoring or measuring leg information from various sensors. The sensors may measure parameters such as strain, impedance, or capacitance to provide leg information, as well as activity of a patient.

In one example, the disclosure is directed to a wearable device comprising a band configured to couple to a leg of a user, a strain sensor attached to the band and configured to generate strain information indicative of changes in circumference of at least a portion of the leg, an activity sensor attached to the band and configured to generate activity information indicative of one or more of an orientation or a movement of the leg, and a processor configured to receive the strain information and activity information and output user information indicative of the strain information and the activity information.

In another example, the band is an elastic band configured to deform with changes to a circumference of the leg of the user.

In another example, the band is configured to substantially encompass at least one of an ankle of the user or a calf of the user.

In another example, the strain sensor comprises at least one strain gauge.

In another example, the wearable device further includes a plurality of strain sensors disposed at different axial locations along the band, each strain sensor of the plurality of strain sensors configured to generate strain information indicative of a respective axial location along the leg of the user.

In another example, the activity sensor comprises a three-axis accelerometer.

In another example, the processor is configured to correlate the strain information to the activity information and determine, based on the correlation of the strain information to the activity information, a level of vascular disease for the leg of the user.

In another example, the wearable device further includes a user interface configured to present the user information.

In another example, the wearable device further includes a communication module configured to wirelessly transmit the user information to an external device according to a command from the processor.

In another example, a method includes generating, by a strain sensor of a wearable computing device associated with a user, strain information indicative of changes in circumference of at least a portion of a leg of the user, generating, by an activity sensor of the wearable computing device, activity information indicative of one or more of an orientation or a movement of the leg, and outputting, by a processor of the wearable computing device, user information indicative of the strain information and the activity information.

In another example, the method further includes correlating the strain information with the activity information.

In another example, the method further includes determining, based on the correlation of the strain information with the activity information, a level of vascular disease for the user.

In another example, the method further includes determining the level of vascular disease by determining a level of chronic venous insufficiency including determining a baseline venous state based on the correlation of the strain information with the activity information to determine a rest state, determining an active venous state based on the correlation of the strain information and activity information to determine an active state, and comparing the baseline venous state to the active state to determine the level of chronic venous insufficiency.

In another example, determining the level of chronic venous insufficiency comprises estimating at least one of a reflux, an obstruction, and a calf pump efficiency of the leg.

In another example, the method further includes presenting, via a user interface of the wearable device, an indication of the level of chronic venous insufficiency.

In another example, the strain information is indicative of at least one of a blood volume and a blood volume over time.

In another example, the activity information is indicative of at least one of a leg orientation or a leg activity over time.

In another example, a method includes receiving, by one or more processors, strain information from a strain sensor of a wearable device, the strain information indicative of changes in circumference of at least a portion of a leg of a user associated with the wearable device, receiving, by the one or more processors, activity information from an activity sensor of the wearable device, the activity information indicative of one or more of an orientation or a movement of the leg, correlating, by the one or more processors, the strain information to the activity information, determining, based on the correlation of the strain information to the activity information, a level of chronic venous insufficiency for the user, and outputting, by the one or more processors and for display at a display device, the level of chronic venous insufficiency determined from the correlation of the strain information to the activity information.

In another example, the method further includes determining, based on the correlation of the strain information with the activity information, a baseline venous state, wherein determining the level of chronic venous insufficiency comprises determining, based on the baseline venous state and correlation of the strain information with the activity information, the level of chronic venous insufficiency.

In another example, the method further includes displaying, via a display device, an indication of the level of chronic venous insufficiency.

In another example, a method includes receiving, by one or more processors, leg information from a sensor of a wearable device, the leg information indicative of a blood volume of at least a portion of a leg of a user associated with the wearable device, receiving, by the one or more processors, activity information from an activity sensor of the wearable device, the activity information indicative of one or more of an orientation or a movement of the leg, correlating, by the one or more processors, the leg information to the activity information, determining, based on the correlation of the leg information to the activity information, a level of chronic venous insufficiency for the user, and outputting, by the one or more processors and for display at a display device, the level of chronic venous insufficiency determined from the correlation of the leg information to the activity information.

In another example, the method further includes determining, based on the correlation of the leg information with the activity information, a baseline venous state, wherein determining the level of chronic venous insufficiency comprises determining, based on the baseline venous state and correlation of the leg information with the activity information, the level of chronic venous insufficiency.

In another example, the leg information includes impedance information about the leg, and the sensor includes an impedance sensor.

In yet another embodiment, the sensor is selected from the group consisting of a strain sensor, an impedance sensor, and a capacitance sensor.

Embodiments may be implemented to realize one or more of the following features. The wearable device and any associated components may provide real-time monitoring over a period of time, which may give a better understanding of the medical condition over time. The wearable device is easy to use and can be used by a patient outside a physician's office during the patient's daily routine. The wearable device may provide multiple data points. The wearable device is noninvasive. The wearable device and any associated components may be used outside a physician's office, thus not requiring the time of a physician to capture the data. The wearable device may also enable a physician to monitor a patient that has certain risk factors for a vascular disease, thus potentially diagnosing a patient at an early stage.

The details of one or more example are set forth in the accompanying drawings and the description below. Other features will be apparent from the description and drawings, and from the claims.

**BRIEF DESCRIPTION OF DRAWINGS**

FIG. 1 is a conceptual diagram illustrating an example system that includes a wearable device and an external device for monitoring chronic venous insufficiency.

FIG. 2 is a conceptual diagram illustrating an example of the wearable device of FIG. 1.

FIG. 3 is a block diagram of an example wearable device of FIG. 1.

FIG. 4 is a flow diagram that illustrates an example process for generating strain information and activity information by a wearable device.

FIG. 5 is a flow diagram that illustrates an example process for determining a level of chronic venous insufficiency based on received strain information and activity information obtained by a wearable device.

FIG. 6 is a block diagram illustrating an example system that includes a networked server coupled to a wearable device and one or more computing devices via a network.

FIG. 7 is a graph illustrating example changes in superficial venous pressure due to standing and running for individuals having various vascular conditions.

FIG. 8 is a conceptual diagram illustrating an example of a wearable device in accordance with another embodiment.

FIG. 9 is a block diagram of an example wearable device of FIG. 8.

FIG. 10 is a flow diagram that illustrates an example process for generating impedance information and activity information by a wearable device of FIGS. 8-9 in accordance with one embodiment.

FIG. 11 is a flow diagram that illustrates an example process for determining a level of chronic venous insufficiency based on received impedance information and activity information obtained by a wearable device of FIGS. 8-9 in accordance with one embodiment.

## DETAILED DESCRIPTION

This disclosure describes devices, systems, and techniques for monitoring and detecting, as an example, venous disease such as chronic venous insufficiency (CVI). CVI is a condition in which blood does not adequately return from one or more legs of a patient to the heart. Although symptoms can be mild and tolerable by a patient at first, symptom severity may increase over time without treatment. In this manner, CVI can go undiagnosed in many patients and underdiagnosed in a population of patients, such as those without regular access to physicians. While the disclosure describes the devices and methods in relation to CVI, the devices and methods may be utilized to monitor and detect other vascular diseases.

As discussed herein, a wearable device may be configured to obtain patient information indicative of CVI such as various leg information. For example, the wearable device may be configured to be coupled to an ankle, a calf, and/or other portion of a leg of the user (e.g., a patient). The wearable device may include mechanisms for detecting changes in blood volume, such as changes in the circumference of the portion of the leg, impedance, or capacitance, and activity of the leg such as orientation with respect to gravity and/or movement. For example, the wearable device may include one or more strain gauges that generate strain information indicative of changes to the circumference of the portion of the leg (e.g., an ankle or calf) of the user. In other examples, the wearable device may include impedance or capacitance sensors that generate impedance or capacitance information indicating changes in the blood volume in the leg. In addition, the wearable device may include one or more activity sensors that generate activity information indicative of the orientation and/or movement of the leg. The wearable device may output (e.g., present and/or transmit to another device) the generated strain information and activity information with or without at least some analysis of the strain information and activity information.

An ankle, calf, or other body part of a user to which the wearable device described herein may be coupled may not be perfectly round in cross-section. Accordingly, the term "circumference" used herein may refer to a distance around the portion of the user's ankle, calf, or other body part to which the wearable device is coupled.

The wearable device or another device (e.g., an external device or a networked device) may correlate the strain information with the activity information and determine a level of CVI based on the correlation. For example, a processor may correlate the strain information with the activity information based on time to establish which activities and/or orientations of the leg correlate with increased or decreased leg circumference (e.g., resulting from changes in blood volume and muscle contraction within the leg). One or more computing devices may further process the correlated information to generate indications of causes of CVI, such as, but not limited to, blood reflux, obstruction, and/or calf muscle pump efficiency.

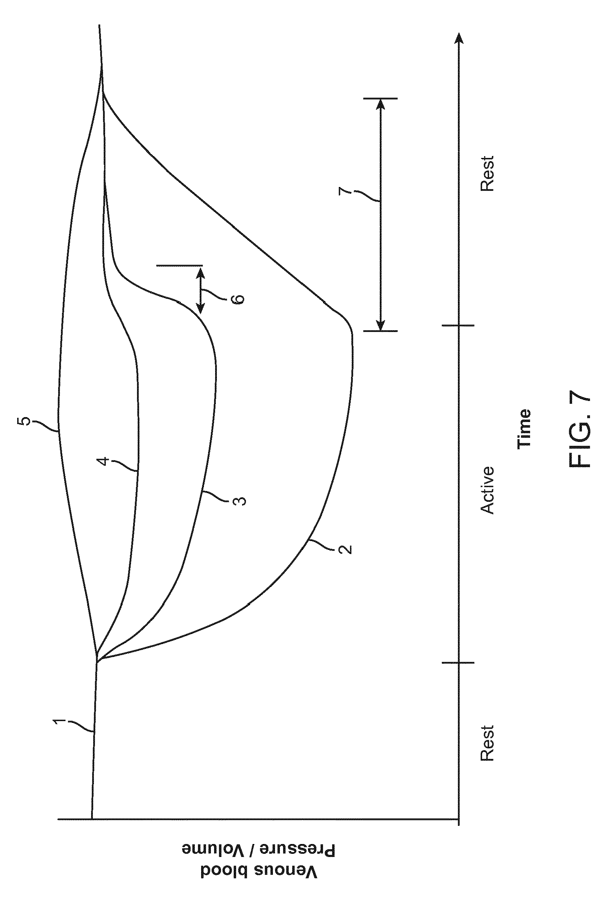

In general, less change in leg circumference when going from sitting to standing and/or upright orientation, or from standing to running may indicate a continual increased blood volume in the leg and a higher probability that the patient has CVI. As can be seen in FIG. 7, for CVI, the amount of change in blood pressure and/or volume from a baseline blood pressure or volume, as well as the rate of change, may be indicative of CVI. For example, curve 1 shows a baseline venous pressure, which may also be correlated to a baseline blood volume. A healthy patient going from a standing position to running will have a greater volume of blood moved from his legs (curve 2) and have a greater recovery time period 7 back to baseline. A patient suffering from superficial CVI will have less blood moved from his legs (curve 3) during running, a patient with deep CVI will have even less blood moved from his legs (curve 4) during running than the patient suffering from superficial CVI, and both patients suffering from CVI will have a quicker recovery time (curve 6) back to baseline because less blood was moved from the legs during running. Further, a patient with a venous obstruction will have a higher volume of blood move into the legs (curve at 5) when moving from a standing position to running. Although the baseline venous blood pressure and/or volume is illustrated as being the same for healthy patients, patients with CVI, and patients with venous obstruction, these baseline pressures and/or volumes may be different for the different types of patients. For example, patients with CVI may have a higher baseline venous blood pressure and/or volume when standing than healthy patients when standing.

Although the devices and techniques described herein are described with respect to an example of CVI detection, the devices and techniques may be applicable to other conditions and diseases. For example, a wearable device described herein may obtain data for diagnosing and tracking the progression of peripheral arterial disease. In addition to, or instead of CVI and peripheral arterial disease, the wearable device may be used to track arterial and/or venous function prior to and/or after surgical or other therapeutic intervention on a leg or other limb of a patient. This may indicate, for example, whether the surgery was successful, such as using a percutaneous transluminal angioplasty (PTA) balloon to open a previously blocked blood vessel.

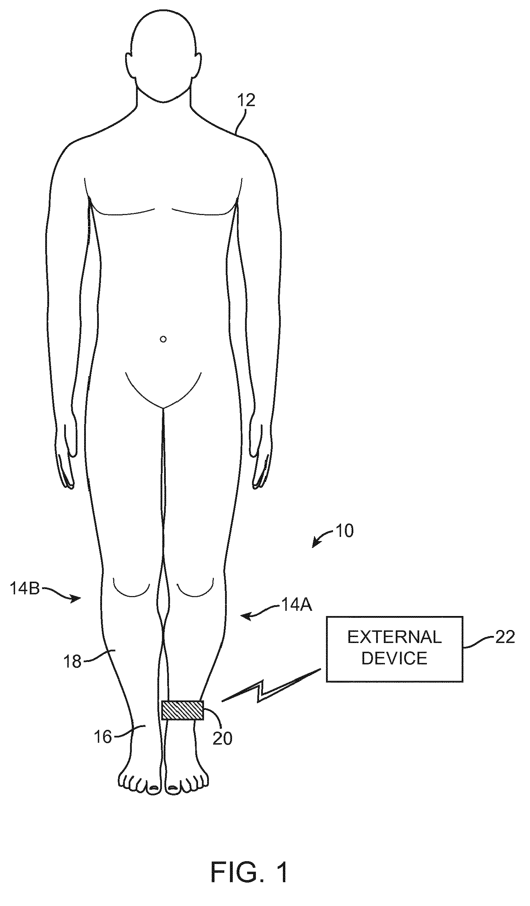

FIG. 1 is a conceptual diagram illustrating an example system 10 for monitoring, for example, CVI. As shown in FIG. 1, system 10 includes a wearable device 20 and an external device 22. The wearable device 20 may be configured to be worn over a portion of a leg 14A or 14B of a user 12. Although the wearable device 20 is shown as being coupled to an ankle 16, the wearable device 20 may alternatively be configured to be worn over at least a portion of a calf 18 or both the ankle 16 and the calf 18. The wearable device 20 may be configured to transfer generated patient information to the external device 22 via any suitable wired or wireless transfer protocols, or both.

The wearable device 20 may include a band configured to couple the wearable device 20 to the leg 14A or 14B of the user 12. The band of wearable device 20 may be an elastic band configured to deform with changes to the circumference of the leg 14A of the user 12. Wearable device 20 may stretch enough to be slid over a foot of the user 12 or include a fastening device that enables the band to be wrapped around the ankle 16 and fastened. In other examples, the band may be ridged with one or more elastic sections that enables deformation in the wearable device 20. Alternatively, the wearable device 20 may be adhered to at least a circumferential portion of the ankle 16 without completely encircling the ankle.

The wearable device 20 may include a strain sensor configured to generate strain information indicative of changes in circumference of at least a portion (e.g., the ankle 16) of the leg 14A. The strain sensor (e.g., a strain gauge) may be attached to at least a portion of the band to sense deformation in the band as the portion of the leg 14A increases or decreased in circumference. The stain sensor may include one or several strain sensors. In some examples, multiple stain sensors may be disposed at different circumferential positions around the wearable device 20 and/or at different axial positions along the length of the leg 14A. The strain information is indicative of a blood volume in the leg 14A over time in addition to muscle contractions.

The wearable device 20 may also include one or more activity sensors configured to generate activity information indicative of one or more of an orientation or a movement of the leg. For example, the activity sensor may be a three-axis accelerometer configured to generate a signal indicative of an orientation of the wearable device 20 with respect to gravity and/or movement of the wearable device 20 and the leg 14A. In other examples, the activity sensor may be a single, dual, or six-axis accelerometer, or a one, two, or three axis gyroscope. In this manner, the activity information may be indicative of at least one of a leg orientation or a leg activity over time. Detected changes in acceleration may indicate movement such as walking or running during which calf muscle contraction and relaxation should pump blood through veins and out of the legs in healthy patients. However, users with CVI may have less blood flow and/or altered blood flow patterns due to damaged vein walls and/or valves or outflow obstruction.

The wearable device 20 may also include one or more processors and/or communication modules. The processor may be configured to output user information indicative of the strain information and the activity information. In some examples, the processor may be configured to analyze the strain information and/or the activity information (e.g., correlate the information and/or determine a level of CVI). In either case, the processor may command a communication module to transmit the raw or processed information to another device (e.g., the external device 22). The communication module may utilize wired or wireless data transmission protocols when communicating with the external device 22.

In some examples, the wearable device 20 may include a user interface configured to present information to the user 12. For example, the user interface may include one or more lights, display devices, speakers, or tactile feedback devices. The processor of the wearable device 20 may instruct the user interface to present information such as an indication of a level of vascular disease, such as CVI, error prompts indicating that wearable device 20 is improperly positioned on the leg 14, a prompt to the user 12 to connect with the external device 22, prompts indicating when the diagnostic monitoring period has been completed, prompts indicating that a memory of the wearable device 20 is full, a low battery power prompt, or any other such information. In addition, or instead, a processor of the wearable device 20 may provide any of this information via the external device 22 by a user interface on the external device 22. Alternatively, in other embodiments, the external device 22 may be a display device configured to display the correlated information from the wearable device 20. For example, the wearable device 20 may correlate the strain and activity information and display the information on a smart phone or tablet as the external device 22.

Although the external device 22 or another computing device different from the wearable device 20 may analyze the strain information and activity information for the level of CVI and/or causes of CVI, in some examples, the wearable device 20 may perform this information processing, or at least a portion of the processing, in other examples. The external device 22 may be a computing device that may receive the strain information from the strain sensor of the wearable device 20 and the activity information from the activity sensor of the wearable device 20. The external device 22 may be configured to correlate the strain information to the activity information and determine, based on the correlation of the strain information to the activity information, a level of chronic venous insufficiency for the user 12. In one embodiment, for example, the external device 22 may be a smart phone or tablet that executes a program, or application, that correlates the strain information to the activity information and displays any relevant information based on that correlation.

For example, in a healthy person without CVI, the circumference of the person's leg may decrease more and quicker with each step the person takes because the cycle with which blood is pumped out of the leg and the contraction and relaxation of the calf muscle can move more blood out of the person's leg than compared to a person with CVI. Similarly, the circumference of the leg of a healthy person will increase more and less quickly than a person with CVI when going from walking or running (e.g., when leg muscles contract and relax) to a standing or sitting position (e.g., when leg muscles are no longer contracting) because more blood volume was removed from the leg of the healthy person during the walking or running activity. It should be noted that a healthy person may have a baseline leg circumference (or leg blood volume) when standing that is smaller than the baseline leg circumference (or leg blood volume) of a person with CVI when standing. Temporally correlating strain and activity information provided by the device 20 may help identify whether such patterns of circumference increase and decrease is seen in the user 12, and, therefore, whether the user 12 may have CVI.

In some examples, external device 22 may be configured to determine a baseline venous state based on the correlation of the strain information with the activity information. For example, a baseline venous state may be the blood volume of the leg 14A when the leg 14A is in a horizontal position (e.g., when the user 12 is lying down) and the leg is at rest. Even for a user with CVI, blood pooling in the legs may be minimal when the legs are in this position. Therefore, external device 22 may compare the circumference and/or estimated blood volume of the baseline venous state with the sensed changes to circumference and/or estimated blood volumes of other leg orientations and/or activities. In users with fully sufficient venous blood flow, the rate and/or amount of changes in leg circumference should be greater between different activity levels. Conversely, users with decreased venous function may develop relatively small changes in leg circumference with a quicker recovery time between different activity levels (e.g., standing versus walking). In this manner, external device 22 may be configured to determine a relative level of CVI based on the baseline venous state and correlation of the strain information with the activity information. As another example, a baseline venous state may be the venous state of the other leg 14B of the user. In addition, or as an alternative, to determining the relative level of CVI based on changes in leg circumference between different activity levels, system 10 may determine the relative level of CVI based on the differences in detected leg circumference between different orientations and/or elevations of a leg of user 12.

In some examples, the external device 22 may be configured to estimate one or more of: a reflux, an obstruction, or a calf pump efficiency of the leg based on the correlated strain information and activity information. The external device 22 may determine reflux and/or obstruction from the rate of blood volume increase in the leg of the user 12 over time. Various algorithms may be utilized to determine the direction and magnitude of average blood volume change between rest and activity. Further, various algorithms may be utilized to determine the rate of change in blood volume on initiation and cessation of activity. In addition, the external device 22 may determine the calf pump efficiency of the leg 14 from the changes and rate of change in circumference in the ankle 16 and/or the calf 18 that occur during calf contractions from walking or heel raises. The external device 22 may determine the level of CVI based on these estimations and/or present these estimations of causes of CVI with the determined level of CVI. The external device 22, or other computing devices, may extract other metrics indicative of CVI from the strain information and activity information in other examples.

Although only one of the wearable device 20 is described in FIG. 1, the system 10 may include two or more of the wearable devices 20. Two or more wearable devices 20 may be disposed at respective locations on one of the legs 14A or 14B to detect circumference changes at each location. In addition, or alternatively, each of the legs 14A and 14B may include one or more wearable device 20. Each wearable device 20 may transmit generated strain information and activity information to the external device 22, and the external device 22, or another computing device, may correlate the information from all wearable devices to establish a more complete dataset for the legs 14A and/or 14B of the user 12.

The external device 22 may output, for display at a display device, the level of chronic venous insufficiency determined from the correlation of the strain information to the activity information. In some examples, the external device 22 may include a user interface (e.g., a display device, lights, speakers, and/or tactile feedback devices) configured to present the indication of the level of CVI and/or other information related to CVI, such as trend information for the correlated strain information and activity information or estimated blood volumes for respective activities or orientations of the leg of the user 12. The external device 22 may also transmit any raw or processed information to other computing devices, servers, or data repositories via one or more networks. In this manner, the external device 22 may facilitate the transmission of user information to remote physicians or other healthcare professionals to diagnose and/or treat the user for CVI.

The system 10 may provide remote monitoring of the user 12 over time with minimal healthcare professional time or other healthcare resources. In this manner, the system 10 and the wearable device 20 may provide a cost effective method for monitoring patients that may have CVI or are at risk for developing CVI. In addition, since the automated monitoring enabled by the wearable device 20 does not generally require a highly trained healthcare professional to conduct the examination, a greater number of patients may have access to this diagnosis method than other typical monitoring techniques. Moreover, the data collected by the wearable device 20 may be more repeatable than subjective measurements taken by a person. In addition, the wearable device 20 is configured to obtain strain information and activity information over a long time period (e.g., days, weeks, or even months) instead of a single measurement at one point in time. In this manner, the wearable device 20 may provide more robust information and indicate trends in the patient condition that can be used to evaluate different aspects of CVI, such as reflux, obstruction, and calf muscle pump effectiveness that otherwise would require separate evaluations.

In addition, a wearable device 20 that may be worn for relatively long periods of time may be useful for monitoring the progress of CVI over time. Because CVI may, in some instances, improve with exercise, the output provided by the wearable device 20 to the user 12 to indicate the status of the CVI (e.g., the relative level of CVI) may provide an incentive for the user 12 to exercise. If the user 12 sees that exercise is providing a positive impact on the user's CVI, the user 12 may be incentivized to be more active.

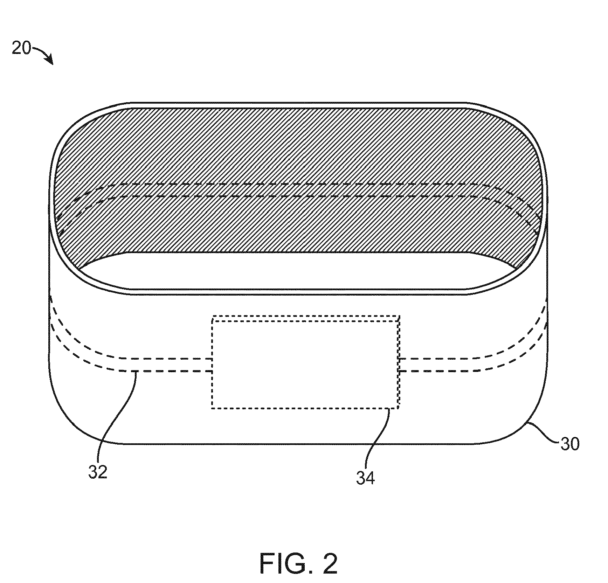

FIG. 2 is a conceptual diagram illustrating an example of the wearable device 20 of FIG. 1. The wearable device 20 may include a band 30, a strain member 32, and a housing 34. The band 30 may be constructed of an elastic material that is deformable to changes in leg circumferences. Although the band 30 is shown without any clasps, other examples of the band may include a clasp that enables the band 30 to be wrapped about the leg and secured. The band 30 may be constructed with minimal thickness to enable the user 12 to place socks, stockings, and pants over the wearable device 20. Further, the band 30 should be configured to provide enough elastic properties to maintain the band 30 in contact with the user's leg, but provide no or minimal compressive forces to the leg that may prevent or minimize the natural blood flow into the leg.

The strain member 32 may be coupled to the strain sensor housed within the housing 34. The strain member 32 may be substantially rigid and coupled at each end to opposite ends of the strain sensor, but compliant enough to be wrapped around the leg 14A/14B. The strain member 32 may be embedded within band 30 or attached to an inner surface or outer surface of the band 30. In this manner, an increase in the circumference of the leg causes each end of the strain member 32 to separate and deform the strain sensor within the housing 34. In other examples, the strain member 32 may be a part of the strain sensor in that deformation of the strain member 32 may cause a change in resistance that is indicative of the circumference change. In some examples, the strain member 32 may not be needed. Instead, one or more strain sensors may be directly coupled to a portion of the elastic band 30.

The housing 34 may be attached to an outer surface of the band 30 or embedded within the band 30 (as shown in FIG. 2). The housing 34 may house at least a portion of a strain sensor, one or more activity sensors, a processor, a communication module, and a power source, as some examples. In addition, the housing 34 may provide a user interface. The housing 34 may be substantially water resistant or waterproof to protect electrical components from water or other fluids during use by the user 12. In some examples, the wearable device 20 may include multiple housings to provide increased flexibility of the wearable device 20 and/or a lower thickness of the housing 34.

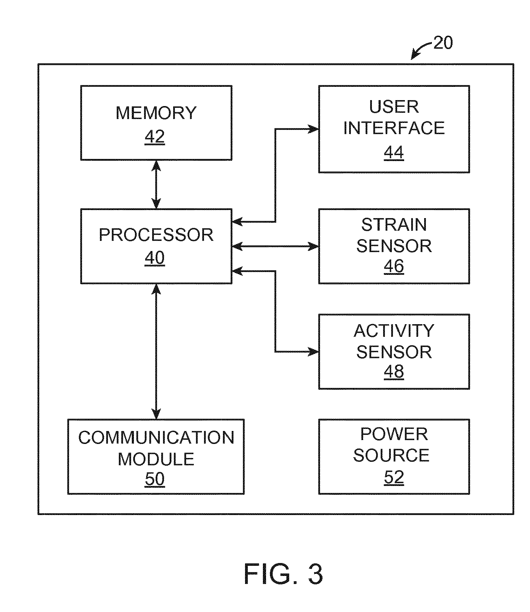

The wearable device 20 may be constructed of different circumference sizes to enable placement with different users and/or at different locations of the leg. The circumference of the band 30 may be selected for a particular user 12 such that when the leg or other body part to which the band 30 is coupled is in a baseline state (e.g., when the user is lying down), the band 30 is in contact with the skin of the patient, but the strain gauge (e.g., a strain gauge of strain sensor 46 shown in FIG. 3) attached to the strain member 32 is not at a maximum strain.

In some examples, the height of the wearable device 20 (e.g., the width of band 30 along the length of the leg) may be small such that the wearable device 20 is similar to a bracelet. In other examples, the wearable device 20 may be constructed with a large height such that the band 30 is similar to a sleeve or extended tube of material. This larger wearable device 20 may extend over ankle 16 and calf 18 and detect circumference at different locations of the leg 14A with respective strain sensors within the same wearable device 20.

FIG. 3 is a block diagram of an example wearable device 20 of FIG. 1. As illustrated in FIG. 3, the wearable device 20 may include a processor 40, a memory 42, a user interface 44, a strain sensor 46, an activity sensor 48, a communication module 50, and a power source 52. The memory 42 may store instructions that, when executed by the processor 40, cause the processor 40 and the wearable device 20 to provide the functionality ascribed to the wearable device 20 throughout this disclosure. For example, the processor 40 may be configured to obtain, store, and/or transmit strain information and activity information.

In general, the wearable device 20 comprises any suitable arrangement of hardware, alone or in combination with software and/or firmware, to perform the techniques attributed to the wearable device 20, and the processor 40, the user interface 44, and the communication module 50 of the wearable device 20. In various examples, the wearable device 20 may include one or more processors, such as one or more: microprocessors, digital signal processors (DSPs), application specific integrated circuits (ASICs), field-programmable gate arrays (FPGAs), or any other equivalent integrated or discrete logic circuitry, as well as any combinations of such components. The wearable device 20 also, in various examples, may include the memory 42, such as a random access memory (RAM), read-only memory (ROM), non-volatile RAM (NVRAM), electrically-erasable programmable ROM (EEPROM), Flash memory, or any other memory device, comprising executable instructions for causing the one or more processors 40 to perform the actions attributed to them. Moreover, although the processor 40 and the communication module 50 are described as separate modules, in some examples, the processor 40 and the communication module 50 are functionally integrated. In some examples, the processor 40 and the communication module 50 correspond to individual hardware units, such as ASICs, DSPs, FPGAs, or other hardware units.

The memory 42 (e.g., a storage device) may store instructions that, when executed by the processor 40, cause the processor 40 and the wearable device 20 to provide the functionality ascribed to the wearable device 20 throughout this disclosure. For example, the memory 42 may include instructions that cause the processor 40 to control the strain sensor 46 and the activity sensor 48 and store obtained strain information and activity information in the memory 42, transmit information to another computing device, or provide any other functionality. In addition, the memory 42 may store data generated by the strain sensor 46 and the activity sensor 48 such as strain information and activity information.

The user interface 44 may include a button or keypad, lights, a speaker for voice commands, a display, such as a liquid crystal (LCD), light-emitting diode (LED), or organic light-emitting diode (OLED). In some examples the display may be a touch screen. The user interface 44 may be configured to display any information related to the functionality of the wearable device 20 such as status information and/or data related to CVI. The wearable device 20 may also receive user input via the user interface 44. The input may be, for example, in the form of pressing a button on a keypad or selecting an icon from a touch screen. In some examples, the wearable device 20 may not include the user interface 44. In another example, the user interface 44 may be an external device, such as a smart phone or tablet, that communicates with the processor 40 by way of the communication module 50.

The strain sensor 46 is an example of, or a component of, the strain member 32 (FIG. 2). The strain sensor 46 may include any electronic components and mechanical component necessary to detect changes in the circumference of a leg of a user. The strain sensor 46 may include one or more of a strain gauge, a resistive fabric, or other component that translates deformation into an electrical signal. The wearable device 20 may include one or more of the strain sensor 46.

The activity sensor 48 may include any electronic components and mechanical components necessary to detect movement and/or orientation of the wearable device 20. The activity sensor 48 may include one or more accelerometer, gyroscope, mercury switch, or any other such device. For example, the activity sensor 48 may include a three-axis accelerometer. Although a single three-axis accelerometer may be used to detect both movement and orientation, activity sensor 48 may include separate components to detect each type of activity. For example, one accelerometer may be configured and calibrated to be sensitive to types of movement typical with legs of the user 12 and another accelerometer configured and calibrated to be sensitive to orientation. In other examples, activity sensor 48 may be a single, dual, or six-axis accelerometer, or a one, two, or three axis gyroscope. In some examples, distinct hardware filters or other signal processing circuitry may be used for the respective accelerometers (or other types of activity sensors) in order to obtain the most accurate data and limit digital processing required by the processor 40.

In some examples, the memory 42 stores data that associates signals from activity sensor 48 with particular user activities. In this way, the processor 40 may determine when the user 12 was engaged in a particular activity (e.g., walking, running, standing upright, and the like), and determine the strain information generated by strain sensor 46 during the time the user 12 was engaged in the activity. The data can include, for example, activity sensor signal templates, threshold values, or any other suitable data that changes as a function of user activity.

The communication module 50 may support wireless communication between the wearable device 20 and the external device 22 (or other computing devices) under the control of the processor 40. The communication module 50 may also be configured to communicate with another computing device via wireless communication techniques, or direct communication through a wired connection. In some examples, communication module 50 may include an antenna, which may take on a variety of forms, such as an internal or external antenna. Examples of local wireless communication techniques that may be employed to facilitate communication between the wearable device 20 and the external device 22 may include RF communication according to the 802.11 or Bluetooth specification sets, or other standard or proprietary telemetry protocols. In this manner, other external devices may be capable of communicating with the wearable device 20 without needing to establish a secure wireless connection.

The power source 52 may include one or more capacitors, batteries, or other energy storage devices. The wearable device 20 may be configured to include electronics that support wired or wireless energy transfer techniques for the power source 52. Alternatively, the power source 52 may not be rechargeable and may or may not be replaceable.

In some examples, the wearable device 20 also includes a feedback mechanism for determining whether the band 30 should be adjusted to reduce the tension in a baseline state. As mentioned above, the wearable device 20 should not be so tight on the leg as to interfere with the natural flow of blood into and out of the leg. For example, the processor 40 may determine that when the user 12 is lying down (as determined by processor 40 by at least detecting an output of the activity sensor 48 associated with the lying down posture state), such that the rate of blood volume change of the leg 14A should be relatively low, the strain sensor 46 should be below a threshold percentage of maximum strain (i.e., a set point). In response to determining, based on the output of strain sensor 46, that the strain sensor 46 is at a strain level greater than or equal to the threshold percentage of maximum strain, the processor 40 may control the user interface 44 to generate an indication that there is too much tension in the band 30 and that either a position of the band 30 should be adjusted or the circumference of the band 30 itself should be adjusted in order to decrease the tension with which the band 30 is engaged with the user 12.

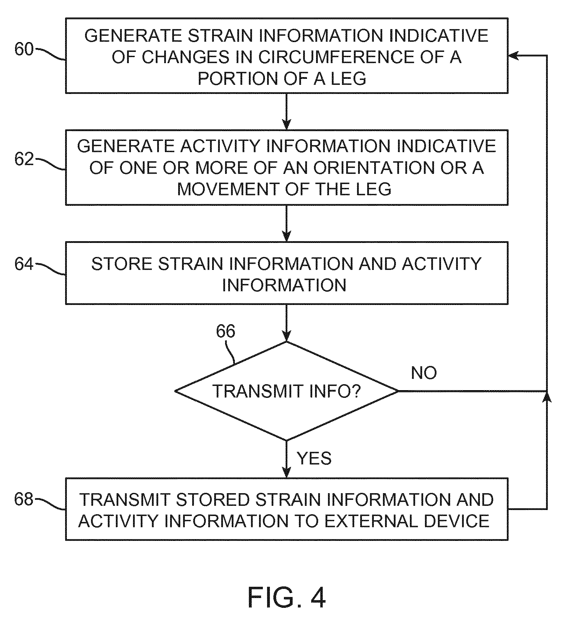

FIG. 4 is a flow diagram that illustrates an example process for generating strain information and activity information by the wearable device 20. As shown in FIG. 4, the strain sensor 46 of the wearable device 20 generates strain information indicative of changes in circumference of a portion of a leg (60). In addition, the activity sensor 48 generates activity information indicative of one or more of an orientation or a movement of a leg of the user (62). The strain sensor 46 and the activity sensor 48 may operate substantially simultaneously such that strain information and activity information is generated for the same period of time.

The processor 40 receives and stores the strain information and the activity information in the memory 42 (64). In some examples, the processor 40 may store the raw data of the strain information and the activity information. In other examples, the processor 40 may perform at least some analysis or processing of the raw data and store the processed data as the stain information and the activity information. For example, the processor 40 may filter the raw data or even temporally correlate the strain information to the activity information.

If the processor 40 receives a request to transmit information from the external device 22 or another device ("YES" branch of block 66), the processor 40 controls the communication module 50 to transmit the stored strain information and activity information to the external device 22 (68) and the wearable device 20 continues to generate new strain information and new activity information (60). If the processor 40 does not receive a request for information ("NO" branch of block 66), the wearable device 22 continues to generate and store information from the strain sensor and activity sensor (60).

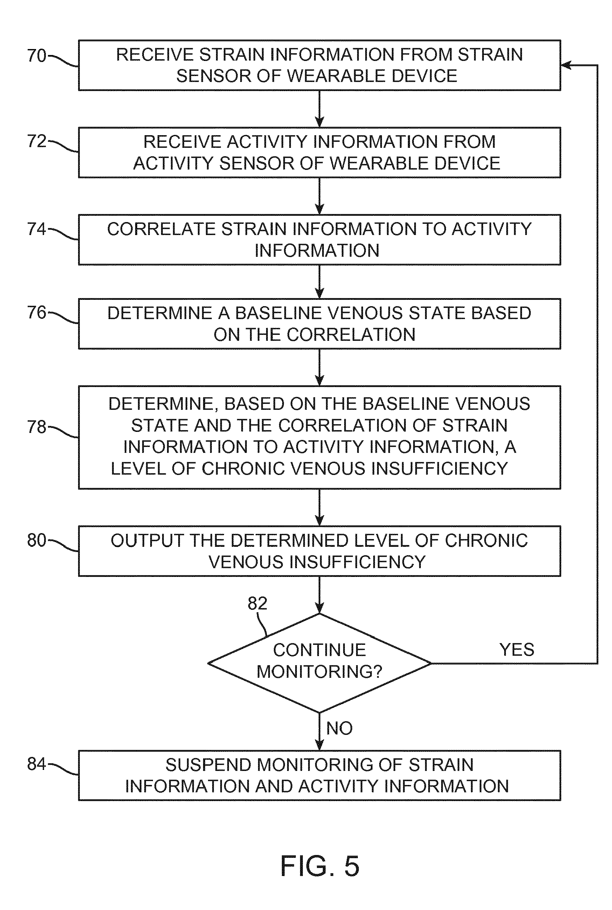

FIG. 5 is a flow diagram that illustrates an example process for determining a level of chronic venous insufficiency based on received strain information and activity information obtained by the wearable device 20. The process of FIG. 5 is described with respect to the external device 22. However, the wearable device 20 may perform some or all of the process of FIG. 5 in other examples.

As shown in FIG. 5, the external device 22 may receive strain information from the strain sensor 46 of the wearable device 20 (70) and activity information from the activity sensor 48 of the wearable device 20 (72). The external device 22 may correlate the strain information to the activity information (74). For example, the external device 22 may temporally correlate the strain and activity information. In some examples, correlation may include establishing circumferential data from the strain information for each of a plurality of different activities and/or orientations from the activity information. The circumferential data may be correlated to show, for example, a percentage change in circumference, absolute change in circumference (e.g., in inches or centimeters), and/or rate of change (e.g., in inches per minute, inches per second, centimeters per minute, etc.).

The external device 22 may then determine a baseline venous state based on the correlation (76). For example, the external device 22 may determine the circumference detected when the leg was horizontal and the rate of change is minimal and establish this value as the baseline venous state. The external device 22 may then determine, based on the baseline venous state and the correlation of the strain information to the activity information, a level of chronic venous insufficiency (CVI) (78). The level may be a binary level such that the user is determined to either have CVI or not have CVI. Alternatively, the level may be indicative of different stages of CVI or a severity of CVI. The level may not directly be a diagnosis of the user. For example, a physician or other healthcare professional may need to review the determined level of CVI to diagnose the user, or determine if further, traditional tests for CVI are needed.

In some examples, the external device 22 may determine estimations of causes of CVI from the strain information and/or activity information. These causes may be reflux, obstruction, and/or calf muscle pump efficiency. For example, the external device 22 may determine that if the strain information indicates the circumference of the ankle 16 of the user 12 increased when the user 12 was engaged in walking or running, a potential cause of the CVI may be an obstruction, such as a clot. As another example, the external device 22 may determine that if the strain information indicates the circumference of the ankle 16 of the user 12 decreased minimally when the user 12 went from standing upright, but not moving, to walking, a potential cause of the CVI may be calf muscle pump issues or reflux.

The external device 22 can output the determined level of CVI (80). This output could be for display on a display device of the external device or the transmission of the level of CVI to another networked device. If the external device 22 has instructions to continue monitoring patient information ("YES" branch of block 82), the external device 22 may continue to receive strain information (70). If the external device 22 receives instructions to stop monitoring the user ("NO" branch of block 82), the external device 22 may suspend monitoring of strain information and activity information from the wearable device 20 (84).

In some examples, the external device 22 may include components similar to those shown in FIG. 3 with respect to the wearable device 20, except the external device 22 may not include the strain sensor 46 and the activity sensor 48. Thus, the one or more processors of external device may perform the technique shown in FIG. 5, and the memory of the external device 22 may store information, such as the baseline venous state of the user, the level of CVI, algorithms for estimating potential causes of the CVI, and the like.

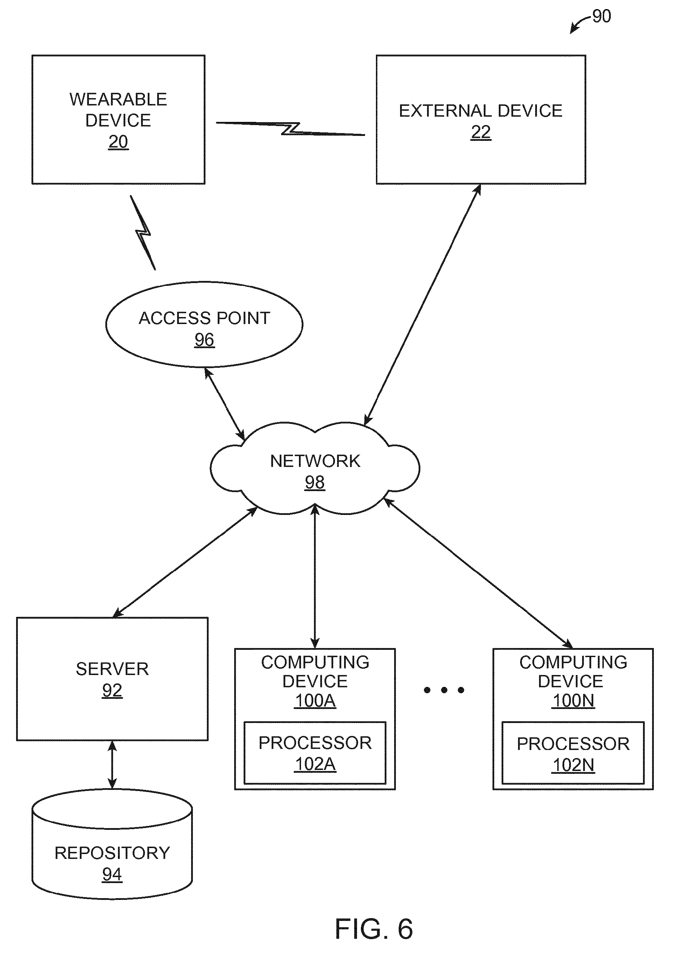

FIG. 6 is a block diagram illustrating an example system 90 that includes a networked server 92 coupled to a wearable device 20 and one or more computing devices 100 via a network 98. In this manner, the wearable device 20 and/or the external device 22 may be coupled to a data distribution network for sharing obtained data from the user 12. As shown in FIG. 6, the server 92 (e.g., a networked external computing device) and one or more computing devices 100A-100N may be coupled to the wearable device 20 and external device shown in FIG. 1 via the network 98. The network 98 may be generally used to transmit strain information, activity information, level of CVI, or any other data between the wearable device 20, the external device 22, the server 92 and/or the computing devices 100.

In some examples, the information transmitted by the wearable device 20 may enable a clinician or other healthcare professional to monitor the user 12 remotely. In some examples, the wearable device 20 may use a telemetry module to communicate with the external device 22 via a first wireless connection, and to communicate with an access point 96 via a second wireless connection, e.g., at different times. In the example of FIG. 6, the access point 96, the external device 22, the server 92 and the computing devices 100A-100N are interconnected, and able to communicate with each other through the network 98. In some cases, one or more of the access point 96, the external device 22, the server 92 and the computing devices 100A-100N may be coupled to the network 98 via one or more wireless connections. The wearable device 20, the external device 22, the server 92, and the computing devices 100A-100N may each comprise one or more processors, such as one or more microprocessors, DSPs, ASICs, FPGAs, programmable logic circuitry, or the like, that may perform various functions and operations, such as those described herein.

The access point 96 may comprise a device that connects to the network 98 via any of a variety of connections, such as telephone dial-up, digital subscriber line (DSL), cable modem connections, and/or cellular networks. In other examples, the access point 96 may be coupled to the network 98 through different forms of connections, including wired or wireless connections. In some examples, the access point 96 may be co-located with the wearable device 20 and may comprise one or more computing devices (e.g., one or more monitoring units) that may perform various functions and operations described herein. For example, the access point 96 may include a home-monitoring unit that is co-located with the user 12 and that may monitor the activity of the wearable device 20. In some examples, the server 92 or the computing devices 100 may control or perform any of the various functions or operations described herein.

In some cases, the server 92 may be configured to provide a secure storage site for archival of strain information, activity information, or other data that has been collected and generated from the wearable device 20. The network 98 may comprise a local area network, wide area network, or global network, such as the Internet. The system of FIG. 6 may be implemented, in some aspects, with general network technology and functionality similar to that provide by the Medtronic CareLink® Network developed by Medtronic, Inc., of Minneapolis, MN.

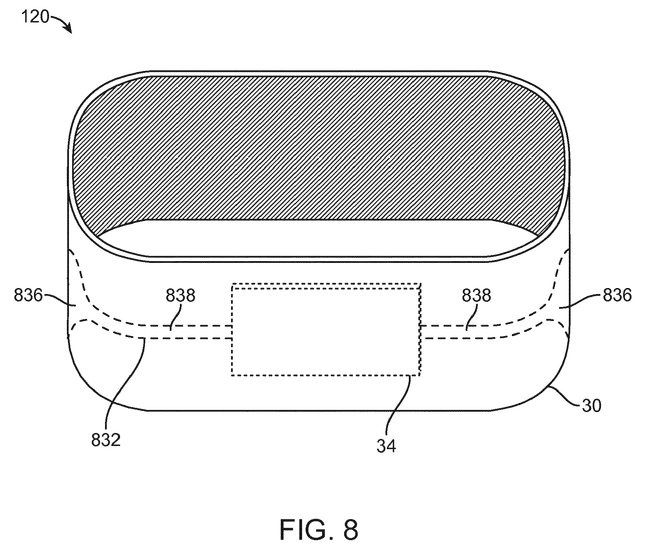

FIG. 8 is a conceptual diagram illustrating an example of a wearable device 120 in accordance with another embodiment. The wearable device 120 of FIG. 8 is similar to the wearable device 20 of FIG. 2 and only the significant differences are discussed below.

In accordance with the embodiment illustrated in FIG. 8, the wearable device 120 may include the band 30, an impedance member 832, and the housing 34. The band 30 may be constructed of an elastic material that is deformable to changes in leg circumferences as discussed above.

The impedance member 832 may be coupled to an impedance sensor housed within the housing 34. The impedance member 832 includes two electrically conductive terminals 836 configured to contact the leg 14A/14B. In one embodiment, the terminals 836 are offset 180° directly opposite from one another on the band 30. For example, the terminals 836 are configured to be located on two opposite sides of the ankle of the leg 14A/14B. However, in other embodiments, terminals 836 are located closer to one another and at an angle less than 180°.

Terminals 836 are electrically connected to the impedance sensor housed within the housing 34 by electrically conductive leads 838.

The impedance member 832 may be embedded within band 30 or attached to an inner surface or outer surface of the band 30 as long as the terminals 836 are exposed or otherwise configured to measure the impedance of the leg 14A/14B.

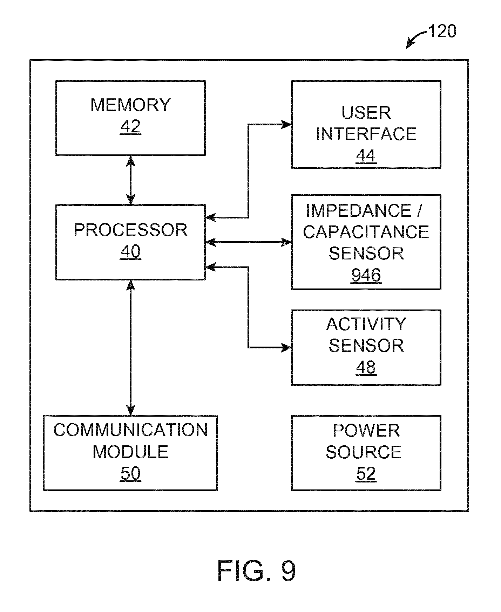

FIG. 9 is a block diagram of an example wearable device 120 of FIG. 8. The block diagram of the wearable device 120 of FIG. 9 is similar to the block diagram of the wearable device 20 of FIG. 3 and only the significant differences are discussed below.

As illustrated in FIG. 9, the wearable device 120 may include the processor 40, the memory 42, the user interface 44, an impedance sensor 946, the activity sensor 48, the communication module 50, and the power source 52. The memory 42 may store instructions that, when executed by the processor 40, cause the processor 40 and the wearable device 120 to provide the functionality ascribed to the wearable device 120 throughout this disclosure. For example, the processor 40 may be configured to obtain, store, and/or transmit impedance information and activity information.

The memory 42 (e.g., a storage device) may store instructions that, when executed by the processor 40, cause the processor 40 and the wearable device 120 to provide the functionality ascribed to the wearable device 120 throughout this disclosure. For example, the memory 42 may include instructions that cause the processor 40 to control the impedance sensor 946 and the activity sensor 48 and store obtained impedance information and activity information in the memory 42, transmit information to another computing device, or provide any other functionality. In addition, the memory 42 may store data generated by the impedance sensor 946 and the activity sensor 48 such as impedance information and activity information.

The impedance sensor 946 is an example of, or a component of, the impedance member 832 (FIG. 8). The impedance sensor 946 may include any electronic components and mechanical component necessary to detect changes in the impedance of a leg of a user. The wearable device 120 may include one or more of the impedance sensor 946.

In one embodiment, impedance is generally the apparent opposition in an electrical circuit to the flow of an alternating current that is analogous to the actual electrical resistance to a direct current, and that is the ratio of effective electromotive force to the effective current. Blood has less impedance than does tissue. Accordingly, as the blood volume of the leg 14A/14B changes, this change is directly measured by measuring the impedance of the leg 14A/14B. More particularly, as the blood volume of the leg 14A/14B goes up, the measured impedance goes down. Conversely, as the blood volume of the leg 14A/14B goes down, the measured impedance goes up. The impedance is measured between the terminals 836.

In one embodiment, the impedance is measured using a 50 kilohertz (KHz) frequency at a 200 micro amp ($\mu$A) current signal, although signals having other frequencies and currents are used in other embodiments.

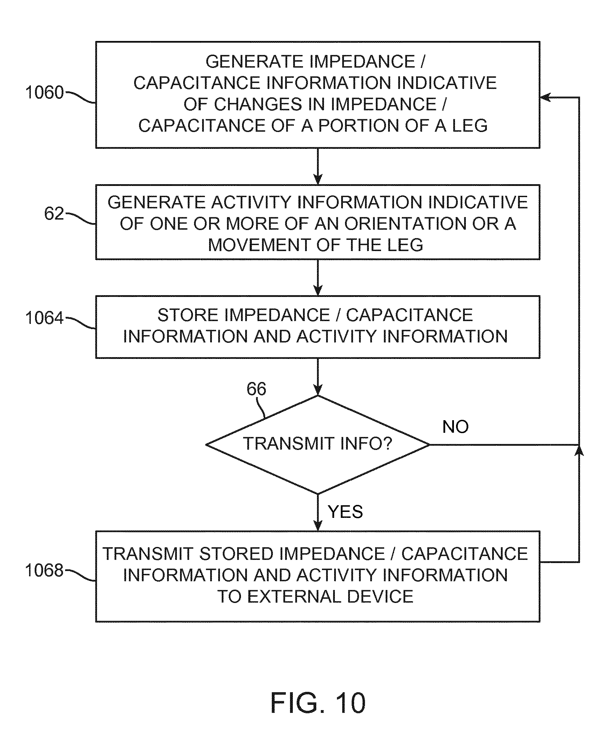

FIG. 10 is a flow diagram that illustrates an example process for generating impedance information and activity information by the wearable device 120 of FIGS. 8-9 in accordance with one embodiment. The flow diagram of FIG. 10 is similar to the flow diagram of FIG. 4 and only the significant differences are discussed below.

As shown in FIG. 10, the impedance sensor 946 of the wearable device 120 generates impedance information indicative of changes in impedance of a portion of a leg (1060). As set forth above, the impedance is inversely related to the blood volume of the leg. In addition, the activity sensor 48 generates activity information indicative of one or more of an orientation or a movement of a leg of the user (62). The impedance sensor 946 and the activity sensor 48 may operate substantially simultaneously such that impedance information and activity information is generated for the same period of time.

The processor 40 receives and stores the impedance information and the activity information in the memory 42 (1064). In some examples, the processor 40 may store the raw data of the impedance information and the activity information. In other examples, the processor 40 may perform at least some analysis or processing of the raw data and store the processed data as the impedance information and the activity information. For example, the processor 40 may filter the raw data or even temporally correlate the impedance information to the activity information.

If the processor 40 receives a request to transmit information from the external device 22 or another device ("YES" branch of block 66), the processor 40 controls the communication module 50 to transmit the stored impedance information and activity information to the external device 22 (1068) and the wearable device 120 continues to generate new impedance information and new activity information (1060). If the processor 40 does not receive a request for information ("NO" branch of block 66), the wearable device 120 continues to generate and store information from the impedance sensor 946 and activity sensor 48 (1060).

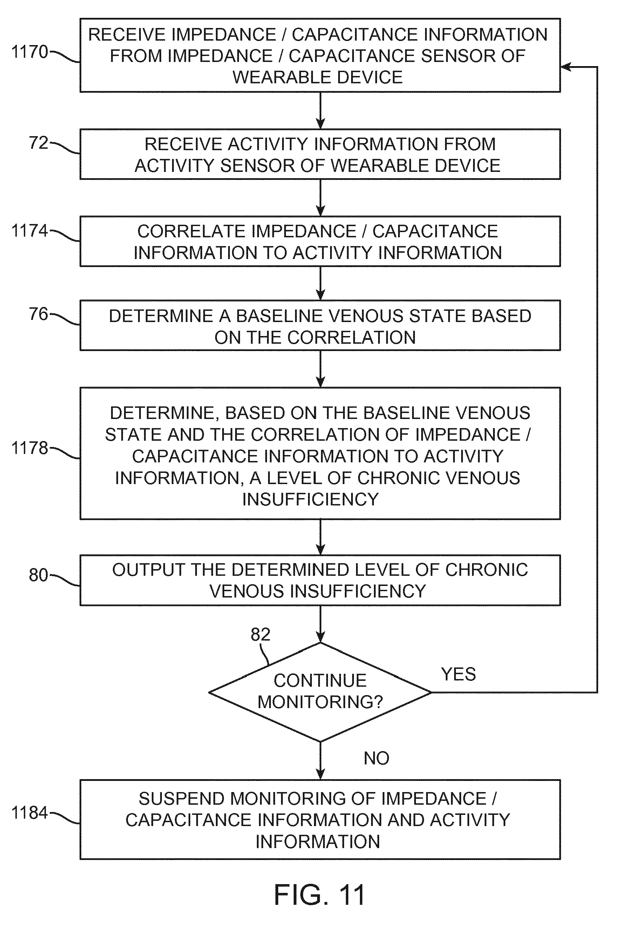

FIG. 11 is a flow diagram that illustrates an example process for determining a level of chronic venous insufficiency based on received impedance information and activity information obtained by the wearable device 120 of FIGS. 8-9 in accordance with one embodiment. The flow diagram of FIG. 11 is similar to the flow diagram of FIG. 5 and only the significant differences are discussed below.

The process of FIG. 11 is described with respect to the external device 22. However, the wearable device 120 may perform some or all of the process of FIG. 11 in other examples.

As shown in FIG. 11, the external device 22 may receive impedance information from the impedance sensor 946 of the wearable device 120 (1170) and activity information from the activity sensor 48 of the wearable device 120 (72). The external device 22 may correlate the impedance information to the activity information (1174). For example, the external device 22 may temporally correlate the impedance and activity information. In some examples, correlation may include establishing impedance data from the impedance information for each of a plurality of different activities and/or orientations from the activity information. The impedance data may be correlated to show, for example, a percentage change in blood volume, blood pressure, a circumference, absolute change in circumference (e.g., in inches or centimeters), and/or rate of change (e.g., in inches per minute, inches per second, centimeters per minute, etc.).

The external device 22 may then determine a baseline venous state based on the correlation (76). For example, the external device 22 may determine the impedance detected when the leg was horizontal and the rate of change is minimal and establish this value as the baseline venous state. The external device 22 may then determine, based on the baseline venous state and the correlation of the impedance information to the activity information, a level of chronic venous insufficiency (CVI) (1178). The level may be a binary level such that the user is determined to either have CVI or not have CVI. Alternatively, the level may be indicative of different stages of CVI or a severity of CVI. The level may not directly be a diagnosis of the user. For example, a physician or other healthcare professional may need to review the determined level of CVI to diagnose the user, or determine if further, traditional tests for CVI are needed.

In some examples, the external device 22 may determine estimations of causes of CVI from the impedance information and/or activity information. These causes may be reflux, obstruction, and/or calf muscle pump efficiency. For example, the external device 22 may determine that if the impedance information indicates the circumference of the ankle 16 of the user 12 increased when the user 12 was engaged in walking or running, a potential cause of the CVI may be an obstruction, such as a clot. As another example, the external device 22 may determine that if the impedance information indicates the circumference of the ankle 16 of the user 12 decreased minimally when the user 12 went from standing upright, but not moving, to walking, a potential cause of the CVI may be calf muscle pump issues or reflux.

The external device 22 can output the determined level of CVI (80). This output could be for display on a display device of the external device or the transmission of the level of CVI to another networked device. If the external device 22 has instructions to continue monitoring patient information ("YES" branch of block 82), the external device 22 may continue to receive impedance information (1170). If the external device 22 receives instructions to stop monitoring the user ("NO" branch of block 82), the external device 22 may suspend monitoring of impedance information and activity information from the wearable device 120 (1184).

In some examples, the external device 22 may include components similar to those shown in FIG. 9 with respect to the wearable device 120, except the external device 22 may not include the impedance sensor 946 and the activity sensor 48. Thus, the one or more processors of external device may perform the technique shown in FIG. 11, and the memory of the external device 22 may store information, such as the baseline venous state of the user, the level of CVI, algorithms for estimating potential causes of the CVI, and the like.

Referring again to FIGS. 8-11, in another embodiment, instead of measuring impedance, capacitance is measured. In accordance with this embodiment, the impedance member 832 and the impedance sensor 946 are a capacitance member 832 and a capacitance sensor 946, respectively, used to measure capacitance of the leg 14A/14B. For example, capacitance is measured between the terminals 836 although more than two terminals can be used, e.g., to provide an array of capacitance information about the geometry/materials in the entire cross-sectional area of the leg 14A/14B.

The measured capacitance is based on the basic equation for capacitance as set forth in equation 1:

$$(1)\ C=E_rE_0A/D,$$

where C is capacitance, $E_r$ is the relative dielectric constant, Eo is the dielectric constant of a vacuum, A is the area of the plates, and D is the distance between the plates. In one embodiment, the plates are the terminals 836.

In accordance with one embodiment, the terminals 836 are placed around the user's extremities, e.g., around the leg 14A/14B. The capacitance between the terminals 836 is measured to give capacitance information about the condition of the leg 14A/14B. More particularly, as the volume, geometry, and material of the leg 14A/14B changes with the change in blood volume, a change in $E_r$ and D occurs. These changes are measured as a change in capacitance that is correlated to the change in blood volume of the leg 14A/14B.

The capacitance information is used in the same manner as the impedance information is used as discussed above and only the significant differences are discussed below. Accordingly, where "impedance/capacitance" is illustrated in FIGS. 9-11, it is to be understood that either impedance or capacitance are represented.

As a general overview, as shown in FIG. 10, the capacitance sensor 946 of the wearable device 120 generates capacitance information indicative of changes in capacitance of a portion of a leg (1060).

The processor 40 receives and stores the capacitance information and the activity information in the memory 42 (1064). If the processor 40 receives a request to transmit information from the external device 22 or another device ("YES" branch of block 66), the processor 40 controls the communication module 50 to transmit the stored capacitance information and activity information to the external device 22 (1068) and the wearable device 120 continues to generate new capacitance information and new activity information (1060). If the processor 40 does not receive a request for information ("NO" branch of block 66), the wearable device 22 continues to generate and store information from the capacitance sensor 946 and activity sensor 48 (1060).

As shown in FIG. 11, the external device 22 may receive capacitance information from the capacitance sensor 946 of the wearable device 120 (1170) and activity information from the activity sensor 48 of the wearable device 120 (72). The external device 22 may correlate the capacitance information to the activity information (1174). For example, the external device 22 may temporally correlate the capacitance and activity information. In some examples, correlation may include establishing capacitance data from the capacitance information for each of a plurality of different activities and/or orientations from the activity information. The capacitance data may be correlated to show, for example, a percentage change in blood volume, blood pressure, a circumference, absolute change in circumference (e.g., in inches or centimeters), and/or rate of change (e.g., in inches per minute, inches per second, centimeters per minute, etc.). In any of the embodiments disclosed herein, blood pressure (venous pressure) can be the target for monitoring, and the measured blood volume is well correlated with the blood pressure.

The external device 22 may then determine a baseline venous state based on the correlation (76). For example, the external device 22 may determine the capacitance detected when the leg was horizontal and the rate of change is minimal and establish this value as the baseline venous state. The external device 22 may then determine, based on the baseline venous state and the correlation of the capacitance information to the activity information, a level of chronic venous insufficiency (CVI) (1178).

In some examples, the external device 22 may determine estimations of causes of CVI from the capacitance information and/or activity information. The external device 22 can output the determined level of CVI (80). This output could be for display on a display device of the external device or the transmission of the level of CVI to another networked device. If the external device 22 has instructions to continue monitoring patient information ("YES" branch of block 82), the external device 22 may continue to receive capacitance information (1170). If the external device 22 receives instructions to stop monitoring the user ("NO" branch of block 82), the external device 22 may suspend monitoring of capacitance information and activity information from the wearable device 120 (1184).

In some examples, the external device 22 may include components similar to those shown in FIG. 9 with respect to the wearable device 120, except the external device 22 may not include the capacitance sensor 946 and the activity sensor 48. Thus, the one or more processors of external device may perform the technique shown in FIG. 11, and the memory of the external device 22 may store information, such as the baseline venous state of the user, the level of CVI, algorithms for estimating potential causes of the CVI, and the like.

Although strain information, impedance information, and capacitance information is discussed above, generally, strain information, impedance information, and capacitance information are all examples of leg information about the condition of the leg used as disclosure herein.

The disclosure contemplates computer-readable storage media comprising instructions to cause a processor to perform any of the functions and techniques described herein. The computer-readable storage media may take the example form of any volatile, non-volatile, magnetic, optical, or electrical media, such as a RAM, ROM, NVRAM, EEPROM, or flash memory that is tangible. The computer-readable storage media may be referred to as non-transitory. A programmer, such as patient programmer or clinician programmer, or other computing device may also contain a more portable removable memory type to enable easy data transfer or offline data analysis.

The techniques described in this disclosure, including those attributed to the wearable device 20, 120, the external device 22, the networked server 92, or the computing devices 100, and various constituent components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, embodied in programmers, such as clinician or patient programmers, stimulators, remote servers, or other devices. The term "processor" or "processing circuitry" may generally refer to any of the foregoing logic circuitry, alone or in combination with other logic circuitry, or any other equivalent circuitry.

Such hardware, software, or firmware may be implemented within the same device or within separate devices to support the various operations and functions described in this disclosure. For example, any of the techniques or processes described herein may be performed within one device or at least partially distributed amongst two or more devices, such as between wearable device 20, 120 and external device 22. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The techniques described in this disclosure may also be embodied or encoded in an article of manufacture including a computer-readable storage medium encoded with instructions. Instructions embedded or encoded in an article of manufacture including a computer-readable storage medium encoded, may cause one or more programmable processors, or other processors, to implement one or more of the techniques described herein, such as when instructions included or encoded in the computer-readable storage medium are executed by the one or more processors. Example computer-readable storage media may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or any other computer readable storage devices or tangible computer readable media. The computer-readable storage medium may also be referred to as one or more storage devices.

In some examples, a computer-readable storage medium comprises non-transitory medium. The term "non-transitory" may indicate that the storage medium is not embodied in a carrier wave or a propagated signal. In certain examples, a non-transitory storage medium may store data that can, over time, change (e.g., in RAM or cache).

Various examples have been described herein. Any combination of the described operations or functions is contemplated. These and other examples are within the scope of the following claims.

**Claims**

1. A wearable device (20) comprising:

a band (30) configured to couple to a leg (14A) of a user;
a strain sensor (46) attached to the band and configured to generate strain information indicative of changes in circumference of at least a portion of the leg;
an activity sensor (48) attached to the band and configured to generate activity information indicative of one or more of an orientation or a movement of the leg; and
a processor (40) configured to receive the strain information and activity information and output user information indicative of the strain information and the activity information.

2. The wearable device of claim 1, wherein the band is an elastic band configured to deform with changes to a circumference of the leg of the user.

3. The wearable device of claim 1, wherein the band is configured to substantially encompass at least one of an ankle (16) of the user or a calf (18) of the user.

4. The wearable device of any one of claims 1-3, wherein the strain sensor comprises at least one strain gauge.

5. The wearable device of any one of claims 1-3, further comprising a plurality of strain sensors disposed at different axial locations along the band, each strain sensor of the plurality of strain sensors being configured to generate strain information indicative of a respective axial location along the leg of the user.

6. The wearable device of any one of claims 1-3, wherein the activity sensor comprises a three-axis accelerometer.

7. The wearable device of any one of claims 1-3, wherein the processor is configured to correlate the strain information to the activity information and determine, based on the correlation of the strain information to the activity information, a level of vascular disease for the leg of the user.

8. The wearable device of any one of claims 1-3, further comprising a user interface (44) configured to present the user information.

9. The wearable device of any one of claims 1-3, further comprising a communication module (50) configured to wirelessly transmit the user information to an external device (22) according to a command from the processor.

10. A method comprising:

generating (60), by a strain sensor (46) of a wearable computing device (20) associated with a user (12), strain information indicative of changes in circumference of at least a portion of a leg (14A) of the user;
generating (62), by an activity sensor (48) of the wearable computing device, activity information indicative of one or more of an orientation or a movement of the leg; and
outputting (68), by a processor (40) of the wearable computing device, user information indicative of the strain information and the activity information.

11. The method of claim 10, further comprising correlating (74) the strain information with the activity information.

12. The method of claim 11, further comprising determining (78), based on the correlation of the strain information with the activity information, a level of vascular disease for the user.

13. The method of claim 12, wherein determining the level of vascular disease comprises determining a level of chronic venous insufficiency comprising:

determining (76) a baseline venous state based on the correlation of the strain information with the activity information to determine a rest state; determining (74) an active venous state based on the correlation of the strain information and activity information to determine an active state; and comparing (78) the baseline venous state to the active state to determine the level of chronic venous insufficiency.

14. The method of claim 13, wherein determining the level of chronic venous insufficiency comprises estimating at least one of a reflux, an obstruction, and a calf pump efficiency of the leg.

15. The method of claim 12, further comprising presenting (80), via a user interface (44) of the wearable device, an indication of the level of chronic venous insufficiency.

16. The method of any one of claims 10-13, wherein the strain information is indicative of at least one of a blood volume and a blood volume over time.

17. The method of any one of claims 10-13, wherein the activity information is indicative of at least one of a leg orientation or a leg activity over time.

18. A method comprising:

receiving (1170), by one or more processors (40), leg information from a sensor (946) of a wearable device (120), the leg information indicative of a blood volume of at least a portion of a leg (14A) of a user (12) associated with the wearable device; receiving (72), by the one or more processors, activity information from an activity sensor (48) of the wearable device, the activity information indicative of one or more of an orientation or a movement of the leg; correlating (1174), by the one or more processors, the leg information to the activity information; determining (1178), based on the correlation of the leg information to the activity information, a level of chronic venous insufficiency for the user; and outputting (80), by the one or more processors and for display at a display device (22), the level of chronic venous insufficiency determined from the correlation of the leg information to the activity information.

19. The method of claim 18, further comprising determining (76), based on the correlation of the leg information with the activity information, a baseline venous state, wherein determining the level of chronic venous insufficiency comprises determining (1178), based on the baseline venous state and correlation of the leg information with the activity information, the level of chronic venous insufficiency.

20. The method of any one of claims 18-19, wherein the leg information comprises impedance information about the leg, the sensor comprising an impedance sensor (946).

21. The method of any one of claims 18-20, wherein the sensor is selected from the group consisting of a strain sensor (46), an impedance sensor (946), and a capacitance sensor (946).

FIG. 1

FIG. 2

FIG. 3

```
              ┌────────────────────────────────────────────────┐
   60──┐      │  GENERATE STRAIN INFORMATION INDICATIVE          │◄──┐
        └─────│  OF CHANGES IN CIRCUMFERENCE OF A                │   │
              │  PORTION OF A LEG                                │   │
              └────────────────────────────────────────────────┘   │
                                  │                                  │
                                  ▼                                  │
              ┌────────────────────────────────────────────────┐   │
   62──┐      │  GENERATE ACTIVITY INFORMATION INDICATIVE        │   │
        └─────│  OF ONE OR MORE OF AN ORIENTATION OR A           │   │
              │  MOVEMENT OF THE LEG                             │   │
              └────────────────────────────────────────────────┘   │
                                  │                                  │
                                  ▼                                  │
              ┌────────────────────────────────────────────────┐   │
   64──┐      │  STORE STRAIN INFORMATION AND ACTIVITY           │   │
        └─────│  INFORMATION                                     │   │
              └────────────────────────────────────────────────┘   │
                  66                │                                │
                    ╲              ▼           NO                    │
                     ◄─────────────────────────────────────────────┤
                     ╱  TRANSMIT INFO?  ╲                           │
                     ╲                  ╱                           │
                       ╲──────────────╱                            │
                            │ YES                                   │
                            ▼                                       │
              ┌────────────────────────────────────────────────┐   │
   68──┐      │  TRANSMIT STORED STRAIN INFORMATION AND          │───┘
        └─────│  ACTIVITY INFORMATION TO EXTERNAL DEVICE         │
              └────────────────────────────────────────────────┘
```

FIG. 4

70 — RECEIVE STRAIN INFORMATION FROM STRAIN SENSOR OF WEARABLE DEVICE

72 — RECEIVE ACTIVITY INFORMATION FROM ACTIVITY SENSOR OF WEARABLE DEVICE

74 — CORRELATE STRAIN INFORMATION TO ACTIVITY INFORMATION

76 — DETERMINE A BASELINE VENOUS STATE BASED ON THE CORRELATION

78 — DETERMINE, BASED ON THE BASELINE VENOUS STATE AND THE CORRELATION OF STRAIN INFORMATION TO ACTIVITY INFORMATION, A LEVEL OF CHRONIC VENOUS INSUFFICIENCY

80 — OUTPUT THE DETERMINED LEVEL OF CHRONIC VENOUS INSUFFICIENCY

82 — CONTINUE MONITORING?

YES

NO

84 — SUSPEND MONITORING OF STRAIN INFORMATION AND ACTIVITY INFORMATION

FIG. 5

90

WEARABLE
DEVICE
20

EXTERNAL DEVICE
22

ACCESS POINT
96

NETWORK
98

SERVER
92

REPOSITORY
94

COMPUTING
DEVICE
100A

PROCESSOR
102A

• • •

COMPUTING
DEVICE
100N

PROCESSOR
102N

FIG. 6

FIG. 7

EP 3 081 162 A2

FIG. 8

120

```
┌──────────────────────────────────────────────────────┐
│                                                        │
│  ┌─────────────────┐        ┌─────────────────┐        │
│  │     MEMORY      │        │      USER        │        │
│  │       42        │        │   INTERFACE      │        │
│  │                 │        │       44         │        │
│  └────────┬────────┘        └─────────────────┘        │
│           │                                            │
│           │                 ┌─────────────────┐        │
│           │                 │  IMPEDANCE /     │        │
│  ┌────────┴────────┐        │  CAPACITANCE     │        │
│  │   PROCESSOR     │◄──────►│    SENSOR        │        │
│  │       40        │        │      946         │        │
│  │                 │        └─────────────────┘        │
│  └────────┬────────┘                                   │
│           │                 ┌─────────────────┐        │
│           │                 │   ACTIVITY       │        │
│           │                 │    SENSOR        │        │
│           │                 │      48          │        │
│  ┌────────┴────────┐        └─────────────────┘        │
│  │ COMMUNICATION   │                                    │
│  │    MODULE       │        ┌─────────────────┐        │
│  │      50         │        │    POWER         │        │
│  └─────────────────┘        │   SOURCE         │        │
│                             │      52          │        │
│                             └─────────────────┘        │
│                                                        │
└──────────────────────────────────────────────────────┘
```

FIG. 9

```
                    ┌──────────────────────────────────────────┐
                    │        GENERATE IMPEDANCE /              │
                    │  CAPACITANCE INFORMATION INDICATIVE     │
        1060 ──     │      OF CHANGES IN IMPEDANCE /          │ ◄──┐
                    │  CAPACITANCE OF A PORTION OF A LEG      │    │
                    └──────────────────────────────────────────┘    │
                                     │                              │
                                     ▼                              │
                    ┌──────────────────────────────────────────┐    │
                    │  GENERATE ACTIVITY INFORMATION INDICATIVE │    │
         62 ──      │  OF ONE OR MORE OF AN ORIENTATION OR A    │    │
                    │       MOVEMENT OF THE LEG                 │    │
                    └──────────────────────────────────────────┘    │
                                     │                              │
                                     ▼                              │
                    ┌──────────────────────────────────────────┐    │
        1064 ──     │     STORE IMPEDANCE / CAPACITANCE         │    │
                    │  INFORMATION AND ACTIVITY INFORMATION     │    │
                    └──────────────────────────────────────────┘    │
                                     │                              │
                66                   ▼                              │
                          ◇──────────────────◇      NO             │
                         ◇   TRANSMIT INFO?    ◇ ─────────────────►│
                          ◇──────────────────◇                     │
                                     │ YES
                                     ▼
                    ┌──────────────────────────────────────────┐
                    │ TRANSMIT STORED IMPEDANCE / CAPACITANCE   │
        1068 ──     │  INFORMATION AND ACTIVITY INFORMATION     │
                    │        TO EXTERNAL DEVICE                 │
                    └──────────────────────────────────────────┘
```

FIG. 10

RECEIVE IMPEDANCE / CAPACITANCE INFORMATION FROM IMPEDANCE / CAPACITANCE SENSOR OF WEARABLE DEVICE — 1170

RECEIVE ACTIVITY INFORMATION FROM ACTIVITY SENSOR OF WEARABLE DEVICE — 72

CORRELATE IMPEDANCE / CAPACITANCE INFORMATION TO ACTIVITY INFORMATION — 1174

DETERMINE A BASELINE VENOUS STATE BASED ON THE CORRELATION — 76

DETERMINE, BASED ON THE BASELINE VENOUS STATE AND THE CORRELATION OF IMPEDANCE / CAPACITANCE INFORMATION TO ACTIVITY INFORMATION, A LEVEL OF CHRONIC VENOUS INSUFFICIENCY — 1178

OUTPUT THE DETERMINED LEVEL OF CHRONIC VENOUS INSUFFICIENCY — 80

CONTINUE MONITORING? — 82

YES

NO

SUSPEND MONITORING OF IMPEDANCE / CAPACITANCE INFORMATION AND ACTIVITY INFORMATION — 1184

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 62137305 A